# EUROPEAN PATENT APPLICATION

(11) **EP 4 205 690 A1**
(43) Date of publication of application: **05.07.2023**
(21) Application number: 22160967.0
(22) Date of filing: 09.03.2022
(51) Int. Cl.: A61B 90/00, A61B 34/10, A61B 34/20

(54) **ENDOBRONCHIAL PROBE TRACKING**

(30) Priority: 30.12.2021 US 202163294892 P
(71) Applicant: Koninklijke Philips N.V., 5656 AG Eindhoven (NL)
(72) Inventor: LEE, Brian C, Eindhoven (NL); SINHA, Ayushi, Eindhoven (NL); VARBLE, Nicole, Eindhoven (NL)
(74) Representative: Philips Intellectual Property & Standards

(57) **Abstract**

The present invention relates to bronchial interventions. In order to provide facilitated navigation support with improved accuracy, a device (10) for endobronchial probe tracking is provided. The device comprises a data input (12); a data processor (14) and an output interface (16). The data input is configured to provide a 3D model of an area of interest of a subject; to provide intraoperatively acquired 2D X-ray projection data of the area of interest of the subject; and to provide endobronchially acquired intraoperative image data of at least a part of the area of interest of the subject. The data processor is configured to track a probe of an interventional device, which probe is acquiring the image data, in the 2D X-ray projection data; to register the 3D model and the 2D X-ray projection data; to register the 3D model and the endobronchially acquired intraoperative image data; and to generate tracking information for the probe of the interventional device in relation to a target location within the area of interest. The output interface is configured to provide the generated tracking information.

## Description

### FIELD OF THE INVENTION

The present invention relates to bronchial interventions and relates in particular to a device for endobronchial probe tracking, to a system for bronchial intervention procedures and to a method for tracking of an endobronchial probe.

### BACKGROUND OF THE INVENTION

Endoscopic techniques are becoming increasingly popular in many surgical disciplines and are becoming the standard of care for diagnostic lung nodule biopsies. An example for a biopsy is a CT-guided percutaneous transthoracic needle aspiration, where a biopsy needle is introduced through the thoracic wall into the lesion to be sampled. Another example is known as transbronchial needle aspiration (TBNA) that aims to navigate a surgical tool through the trachea into the main airways of the lung, then into the narrower peripheral airways. WO 2021/122344 A1 relates to navigating bronchial pathways. In a further example, the surgical tool travels along a pathway determined by the pre-operative 3D CT scan and the 3D location of the detected lesion. However, navigating through the narrow peripheral airways is difficult.

### SUMMARY OF THE INVENTION

There may thus be a need to provide facilitated navigation support with improved accuracy.

The object of the present invention is solved by the subject-matter of the independent claims; further embodiments are incorporated in the dependent claims. It should be noted that the following described aspects of the invention apply also for the device for endobronchial probe tracking, for the system for bronchial intervention procedures and for the method for tracking of an endobronchial probe.

According to the present invention, a device for endobronchial probe tracking is provided. The device comprises a data input, a data processor and an output interface. The data input is configured to provide a 3D model of an area of interest of a subject. The data input is also configured to provide intraoperatively acquired 2D X-ray projection data of the area of interest of the subject. The data input is further configured to provide endobronchially acquired intraoperative image data of at least a part of the area of interest of the subject. The data processor is configured to track a probe of an interventional device, which probe is acquiring the image data, in the 2D X-ray projection data. The data processor is configured to register the 3D model and the 2D X-ray projection data. The data processor is further configured to register the 3D model and the endobronchially acquired intraoperative image data. The data processor is also configured to generate tracking information for the probe of the interventional device in relation to a target location within the area of interest. The output interface is configured to provide the generated tracking information.

As an effect, the user can be provided with navigation feedback relating to the actual position of the device.

According to an example, in a first option, the provided endobronchially acquired intraoperative image data is provided as endobronchially acquired intraoperative ultrasound image data. In a second option, in addition or alternatively, the provided endobronchially acquired intraoperative image data is provided as endobronchially acquired intraoperative bronchoscopic image data.

According to an example, the tracking information comprises an overlay of an estimated position of the probe onto the 3D model.

According to an example, to track the probe of the interventional device, the data processor is configured to determine a projection angle of the 2D X-ray projection in relation to the 3D model.

According to an example, to register the 3D model and the 2D X-ray projection data, the data processor is configured to provide the 3D model with a first notation of a landmark. The data processor is also configured to provide the 2D X-ray projection data with a second notation of the landmark, which first and second notation of the landmark are subject to an alignment.

According to an example, to register the 3D model and the endobronchially acquired image data, the data processor is configured to generate, based on the 3D model, a simulated image for a given position of the probe. The data processor is also configured to compare the simulated image for similarity with the intraoperative image data. In an option, the data processor is also configured to maximize said similarity.

According to an example, to register the 3D model and the image data, the data processor is configured to extract salient features from the image data, and to identify corresponding features in the 3D model.

According to an example, the data input is configured to provide both ultrasound image data and bronchoscopic image data. The data processor is configured to register the bronchoscopic image data with at least one of the group of the 3D model and the 2D X-ray projection data.

According to the present invention, also a system for bronchial intervention procedures is provided. The system comprises a 2D X-ray imaging arrangement, an imaging arrangement for endobronchial imaging and a device for endobronchial probe tracking according to one of the preceding examples. The 2D X-ray imaging arrangement comprises an X-ray source and an X-ray detector configured to generate the 2D X-ray projection data. The imaging arrangement comprises an interventional device with an imaging probe for insertion into the bronchial pathway configured to generate the intraoperative image data.

According to the present invention, also a method for tracking of an endobronchial probe is provided. The method comprises the following steps:
- providing a 3D model of an area of interest of a subject;
- providing intraoperatively acquired 2D X-ray projection data of the area of interest of the subject;
- providing endobronchially acquired intraoperative image data of at least a part of the area of interest of the subject;
- tracking a probe of an interventional device, which probe is acquiring the image data, in the 2D X-ray projection data;
- registering the 3D model and the 2D X-ray projection data;
- registering the 3D model and the endobronchially acquired intraoperative image data;
- generating tracking information for the probe of the interventional device in relation to a target location within the area of interest; and
- providing the generated tracking information.

In another option, a device for endobronchial probe tracking is provided. The device comprises: a data input, a data processor and an output interface. The data input is configured: to provide a 3D model of an area of interest of a subject; and to provide one of the group of intraoperatively acquired 2D X-ray projection data of the area of interest of the subject and endobronchially acquired intraoperative image data of at least a part of the area of interest of the subject. The data processor is configured to track a probe of an interventional device, which probe is acquiring the image data, in the 2D X-ray projection data; to register the 3D model and the one of the group of the 2D X-ray projection data and the 3D model and the endobronchially acquired intraoperative image data. The data processor is also configured to generate tracking information for the probe of the interventional device in relation to a target location within the area of interest. For the generation of the tracking information, in addition to the available image data, one of the group of a last known approximate region from a latest X-ray projection data and a latest endobronchially acquired intraoperative image data is used. The output interface is configured to provide the generated tracking information.

In another option, a method for tracking of an endobronchial probe is provided. The method comprises the following steps:
- providing a 3D model of an area of interest of a subject;
- providing one of the group of intraoperatively acquired 2D X-ray projection data of the area of interest of the subject and endobronchially acquired intraoperative image data of at least a part of the area of interest of the subject;
- tracking a probe of an interventional device, which probe is acquiring the image data, in the 2D X-ray projection data;

- registering the 3D model and the one of the group of the 2D X-ray projection data and the 3D model and the endobronchially acquired intraoperative image data;
- generating tracking information for the probe of the interventional device in relation to a target location within the area of interest; wherein for the generating of the tracking information, in addition to the available image data, one of the group of a last known approximate region from a latest X-ray projection data and a latest endobronchially acquired intraoperative image data is used; and
- providing the generated tracking information.

In a still further option, a method for tracking of an endobronchial probe is provided. The method comprises the following steps:
- providing a 3D model of an area of interest of a subject;
- providing at least one of the group of: i) intraoperatively acquired 2D X-ray projection data of the area of interest of the subject, and ii) endobronchially acquired intraoperative image data of at least a part of the area of interest of the subject;
- analyzing if both of the group of i) intraoperatively acquired 2D X-ray projection data and ii) endobronchially acquired intraoperative image data are provided; and if both are provided performing the steps of:
- tracking a probe of an interventional device, which probe is acquiring the image data, in the 2D X-ray projection data;
- registering the 3D model and the 2D X-ray projection data;
- registering the 3D model and the endobronchially acquired intraoperative image data;
- generating tracking information for the probe of the interventional device in relation to a target location within the area of interest; and
- providing the generated tracking information.

According to an aspect, feedback between the 3D-to-X-ray registration (also referred to as 3D/X-ray registration) and the 3D-to-ultrasound registration (3D/US registration ) is provided. This is provided in the sense that the results of the 3D/X-ray registration reduce the search space for the 3D/US registration. This avoids or at least minimizes situations where a 3D/US registration would be likely to be intractable. This, i.e. the reduction of the search space, then reduces the search space for the 2^{nd} iteration of the 3D/X-ray registration.

According to an aspect, the solutions discussed above are designed to tackle problems where there is imperfect information from multiple intraoperative image sources that, however, may be able to benefit each other. As an example, a large field of view may be provided by one imaging source, but depth is reduced or missing in X-ray, versus small field of view, but with enough information to extract depth in REBUS (radial endobronchial ultrasonography).

According to an aspect, tracking and thus navigation feedback is provided based on different imaging sources in which information of the current image sources is supplemented by spatial information from a model.

As an effect, operation of the system under ultrasound guidance with all other intra-operative image sources turned off is still possible due to updating the estimated location of a tool based on intra-operative ultrasound.

These and other aspects of the present invention will become apparent from and be elucidated with reference to the embodiments described hereinafter.

### BRIEF DESCRIPTION OF THE DRAWINGS

Exemplary embodiments of the invention will be described in the following with reference to the following drawings:
Fig. 1 schematically shows an example of a device for endobronchial probe tracking.
Fig. 2 shows a system for bronchial intervention procedures.
Fig. 3 shows steps of an example of method for tracking of an endobronchial probe.
Fig. 4 shows an example of a workflow.
Figs. 5a and 5b show an example for bifurcation detection framework.

### DETAILED DESCRIPTION OF EMBODIMENTS

Certain embodiments will now be described in greater details with reference to the accompanying drawings. In the following description, like drawing reference numerals are used for like elements, even in different drawings. The matters defined in the description, such as detailed construction and elements, are provided to assist in a comprehensive understanding of the exemplary embodiments. Also, well-known functions or constructions are not described in detail since they would obscure the embodiments with unnecessary detail. Moreover, expressions such as "at least one of', when preceding a list of elements, modify the entire list of elements and do not modify the individual elements of the list.

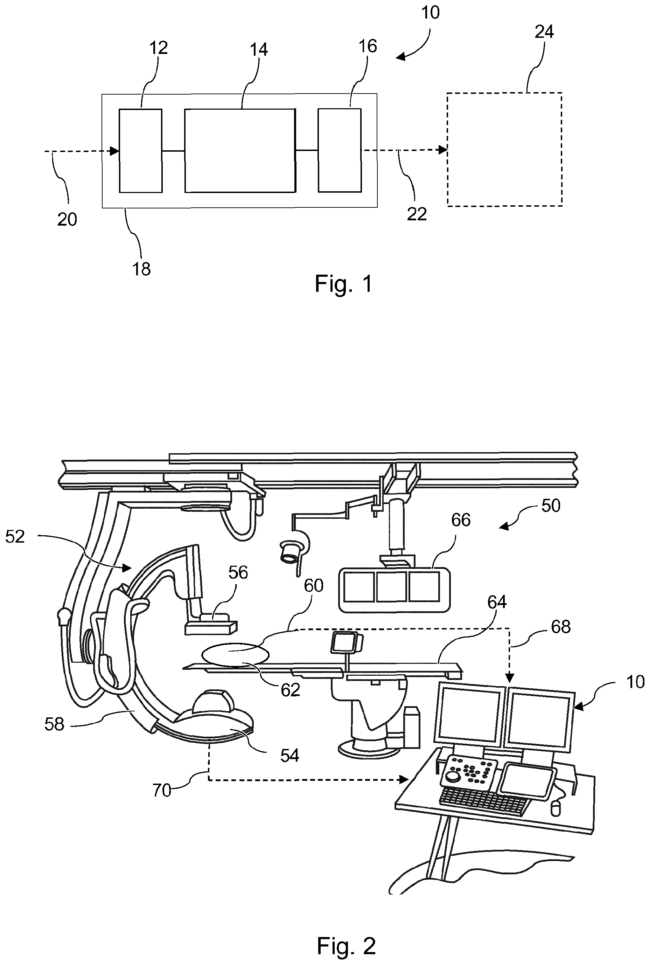

Fig. 1 schematically shows an example of a device 10 for endobronchial probe tracking. The device 10 comprises a data input 12, a data processor 14 and an output interface 16. The data input 12 is configured to provide a 3D model of an area of interest of a subject. The data input 12 is also configured to provide intraoperatively acquired 2D X-ray projection data of the area of interest of the subject. The data input 12 is further configured to provide endobronchially acquired intraoperative image data of at least a part of the area of interest of the subject. The data processor 14 is configured to track a probe of an interventional device, which probe is acquiring the image data, in the 2D X-ray projection data. The data processor 14 is also configured to register the 3D model and the 2D X-ray projection data. The data processor 14 is further configured to register the 3D model and the endobronchially acquired intraoperative image data. The data processor 14 is furthermore configured to generate tracking information for the probe of the interventional device in relation to a target location within the area of interest. The output interface 16 is configured to provide the generated tracking information.

A frame is shown indicating an option according to which the data input 12, the data processor 14 and the output interface 16 are provided in an integrated manner, e.g. in a common housing. In another option, the data input 12, the data processor 14 and the output interface 16 are provided separately.

A first arrow 20 indicates the data supply, i.e. the data input. A second arrow 22 indicates the provision of the generated data, i.e. the data output.

The term "3D model" relates to a data volume representing an anatomical structure in a spatial arrangement. In an example, the 3D model comprises a determined target location. The determination of the target location can be based on previous imaging and/or intervention procedures.

The term "intraoperatively" relates to data acquisition during an intervention like an operation, contrary to "pre-operatively", which relates to data acquisition before an intervention takes place. The intraoperatively acquired image data is acquired as current or real-time image data. The intraoperatively acquired image data can also be referred to as live image data.

The term "2D X-ray projection data" relates to image data generated by projection of X-ray radiation. For example, low-dose fluoroscopic imaging is provided.

The term "endobronchially" relates to placing an imaging probe within the lumen of the bronchus, i.e. the bronchial tube or the bronchia.

The probe is an imaging probe, e.g. an ultrasound imaging probe or an endoscopic camera probe. In an example, the probe comprises a probe tip.

The term "registering" relates to determining the spatial relation of the reference frame of the one imaging coordinate frame, or reference frame like the 2D X-ray projection, with the other imaging coordinate frame, or reference frame, like the 3D model, or the endobronchial imaging. By registering, positions or locations can be transferred from one imaging world into the other imaging world. Registering can also comprise a geometric adaption of the one image data to spatially match into the other image's reference frame.

The term "tracking information" relates to information that relates to the position of the imaging probe, which information can be used for identifying the positional relation of the probe and the target location. The information thus allows to track the probe within the area or region of interest.

The term tracking relates to localizing the probe. Hence, the tracking information can also be referred to as "localization information". In an example, the probe is localized in the 2D X-ray projection data and localization information is generated and provided.

The term "target location" relates to a location within the area or region of interest that has been determined before the procedure. As an example, the target location is a tissue location that will be subject to sample collection, i.e. biopsy.

In a first option, the provided endobronchially acquired intraoperative image data is provided as i) endobronchially acquired intraoperative ultrasound image data.

In a second option, the provided endobronchially acquired intraoperative image data is provided as ii) endobronchially acquired intraoperative bronchoscopic image data.

In an example, the endobronchially acquired intraoperative image data is provided by an imaging device inserted in an endobronchial way to acquire the image data.

Shown as an option in Fig. 1, a display 24 is provided that is configured to present the generated tracking information to a user.

In an example, as a first option, the tracking information comprises an overlay of an estimated position of the probe onto the 3D model.

In an example, as a second option in addition or alternatively, not shown in detail, the tracking information comprises navigation information for the user for directing the probe towards the target location.

In an example, the registering of the 3D model and the 2D X-ray projection data comprises transferring a position of the detected probe from a reference frame of the 2D X-ray projection data into a reference frame of the 3D model. The transferring of the position is based on the registration of the 3D model and the 2D X-ray projection data. For generating the navigation information, a relative displacement between the transferred probe position and a target location in the 3D model is detected and a possible path for the probe to the target location is determined.

In an example, not shown in detail, the 3D model is a subject-specific 3D model.

In an example, the 3D model is an individualized model relating to the area of interest of the subject. The model is subject- or patient-specific.

In another example, the 3D model is generated from CT data of the area of interest of the subject.

In another example, the 3D model is based on a generic model, which is adapted to the particular subject.

In an example, not shown in detail, to track the probe of the interventional device, the data processor is configured to determine a projection angle of the 2D X-ray projection in relation to the 3D model.

In an example, not shown in detail, to register the 3D model and the 2D X-ray projection data, the data processor is configured: to provide the 3D model with a first notation of a landmark; and to provide the 2D X-ray projection data with a second notation of the landmark, which first and second notation of the landmark are subject to an alignment.

In an example, the landmark alignment can also be a "soft" alignment in that the registration could especially penalize solutions in which the two landmarks end up far apart rather than fixing the two points as absolute correspondences.

In an example, not shown in detail, to register the 3D model and the endobronchially acquired image data, the data processor is configured: to generate, based on the 3D model, a simulated image for a given position of the probe; and to compare the simulated image for similarity with the intraoperative image data. The data processor is also configured to determine the probe position that maximizes this similarity.

In a first example, the image data is provided as ultrasound image data. To register the 3D model and the ultrasound image data, the data processor is configured to generate a simulated ultrasound image for a given position and orientation of the probe and to compare the simulated ultrasound image for similarity with the intraoperative ultrasound image data.

In a second example, the image data is provided as bronchoscopic image data. To register the 3D model and the bronchoscopic image data, the data processor is configured to generate a simulated bronchoscopic image for a given position of the probe and to compare the simulated bronchoscopic image for similarity with the intraoperative bronchoscopic image data.

In an example, not shown in detail, to register the 3D model and the image data, the data processor is configured: to extract salient features from the image data; and to identify corresponding features in the 3D model.

In an example, additional information beyond image-based features is detected, such as the approximate distance that the probe has been advanced. For example, the distance between branching vessels is matched against the airway tree to estimate the probe position.

In a first example, to register the 3D model and the ultrasound image data, the data processor is configured to extract salient features from the ultrasound image data and to identify these features in the 3D model.

In a second example, to register the 3D model and the bronchoscopic image data, the data processor is configured to extract salient features from the bronchoscopic image data and to identify these features in the 3D model.

In an example, an estimated probe position is updated by a further registration of the 3D model with updated intraoperatively acquired 2D X-ray projection data. In another example, an estimated probe position is updated by a further registration of the 3D model with updated intraoperatively acquired ultrasound (or bronchoscopic) image data.

In an example, not shown in detail, the data input is configured to provide both ultrasound image data and bronchoscopic image data. The data processor is configured to register the bronchoscopic image data with at least one of the group of the 3D model and the 2D X-ray projection data.

Bronchoscopic imaging is also referred to as bronchoscopy.

In an option, the intraoperative ultrasound image data and the bronchoscopic image data are acquired simultaneously.

In another option, as an update loop, when only one of the group of the X-ray projection data and the endobronchially acquired intraoperative image data is available, the data processor is configured to update the current estimated probe position only, by use of, in addition to the available image data, one of the group of the last known approximate region from the latest X-ray projection data and the latest endobronchially acquired intraoperative image data.

Fig. 2 shows a system 50 for bronchial intervention procedures. The system 50 comprises a 2D X-ray imaging arrangement 52. The 2D X-ray imaging arrangement 52 comprises an X-ray source 54 and an X-ray detector 56 configured to generate the 2D X-ray projection data. As an example, the X-ray source 54 and the X-ray detector 56 are attached to opposite ends of a movably mounted C-arm 58. Further, an imaging arrangement 60 for endobronchial imaging is provided. A subject 62 is indicated on am optional subject support 64. A monitor arrangement 66 is shown together with lighting equipment mounted to a ceiling rail structure. An example of the device 10 for endobronchial probe tracking according to one of the preceding examples is provided. The imaging arrangement 60 for endobronchial imaging is data-connected to the device 10 for endobronchial probe tracking as indicated with a first hashed line 68. Further, also the 2D X-ray imaging arrangement 52 is data-connected to the device for endobronchial probe tracking 10 as indicated with a second hashed line 70. The imaging arrangement 60 comprises an interventional device with an imaging probe for insertion into the bronchial pathway configured to generate the intraoperative image data.

The device for endobronchial probe tracking 10 is shown in the context of a console with different user interfaces, like keyboard, mouse, tablet, control knobs and graphical display.

In a first option, the imaging arrangement for endobronchial imaging is an ultrasound imaging arrangement providing endobronchially acquired intraoperative ultrasound image data.

In a second option, the imaging arrangement for endobronchial imaging is a bronchoscopic imaging arrangement providing endobronchially acquired intraoperative bronchoscopic image data.

The bronchoscopic imaging arrangement is also referred to as bronchoscopy imaging arrangement.

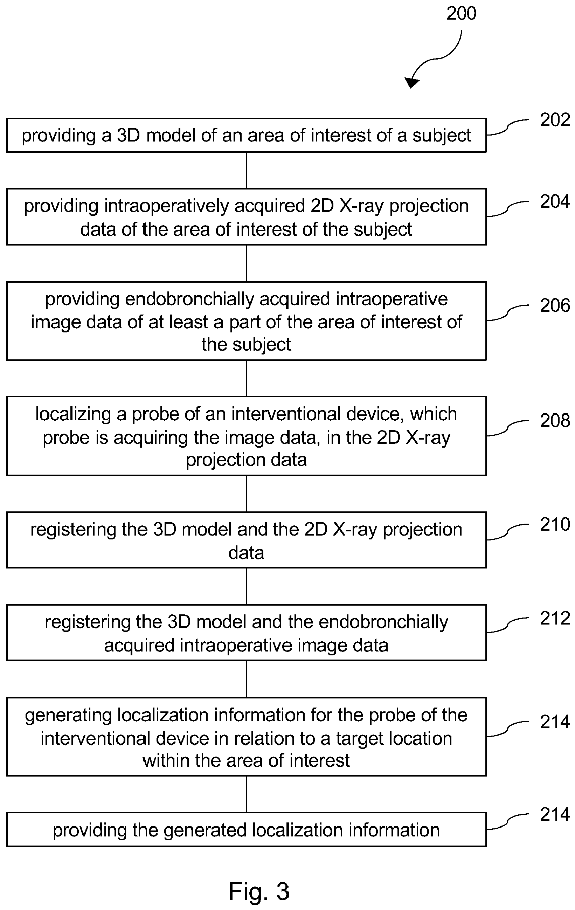

Fig. 3 shows steps of an example of a method 200 for tracking of an endobronchial probe. The method 200 comprises the following steps:
- In a first step 202, a 3D model of an area of interest of a subject is provided.
- In a second step 204, intraoperatively acquired 2D X-ray projection data of the area of interest of the subject is provided.
- In a third step 206, endobronchially acquired intraoperative image data of at least a part of the area of interest of the subject is provided.
- In a fourth step 208, a probe of an interventional device is tracked in the 2D X-ray projection data, which probe is acquiring the image data.
- In a fifth step 210, the 3D model and the 2D X-ray projection data are registered.
- In a sixth step 212, the 3D model and the endobronchially acquired intraoperative image data are registered.
- In a seventh step 214, tracking information for the probe of the interventional device in relation to a target location within the area of interest is generated.
- In an eighth step 216, the generated tracking information is provided.

In an example, the second registration step is initialized based on the registration between 3D model and X-ray in order to improve accuracy, for instance that the first registration step reduces the search space for the second registration step to a small region of interest. In an option, this is provided iteratively.

For example, interaction between the tracking step, i.e. the fourth step 208, and the registration step, i.e. the fifth step 210, is provided. The probe of the interventional device is tracked in the 2D X-ray projection data in order to provide a possible basis for the registration of the 3D model and the 2D X-ray projection data.

In an example, step 212 may include information that is supplied from the output of previous step, i.e. of step 210.

In an example, for detecting, i.e. tracking or localizing the probe, a segmentation is provided to detect the tip location. This leads to an assumption of where the probe position might be located.

The registration is done based on available information. This may comprise a registration based on segmentation result, or based on landmarks, or based on known imaging geometry, or based on fiducial markers and the like.

In a first option, the endobronchially acquired intraoperative image data is provided as ultrasound image data. Hence, a method for tracking an endobronchial probe is provided that comprises the following steps:
- providing a 3D model of an area of interest of a subject;
- providing intraoperatively acquired 2D X-ray projection data of the area of interest of the subject;
- providing endobronchially acquired intraoperative ultrasound image data of at least a part of the area of interest of the subject;
- detecting a probe of an interventional device, which probe is acquiring the ultrasound image data, in the 2D X-ray projection data;
- registering the 3D model and the 2D X-ray projection data;
- registering the 3D model and the ultrasound image data;
- generating tracking information for the ultrasound imaging probe of the interventional device in relation to a target location within the area of interest; and
- providing the generated tracking information.

In a second option, the endobronchially acquired intraoperative image data is provided as bronchoscopic image data. Hence, a method for tracking an endobronchial probe is provided that comprises the following steps:
- providing a 3D model of an area of interest of a subject;
- providing intraoperatively acquired 2D X-ray projection data of the area of interest of the subject;
- providing endobronchially acquired intraoperative bronchoscopic image data of at least a part of the area of interest of the subject;
- detecting a probe of an interventional device, which probe is acquiring the bronchoscopic image data, in the 2D X-ray projection data;
- registering the 3D model and the 2D X-ray projection data;
- registering the 3D model and the bronchoscopic image data;
- generating tracking information for the bronchoscopic imaging probe of the interventional device in relation to a target location within the area of interest; and
- providing the generated tracking information.

In an example, the registering of the 3D model and the 2D X-ray projection data provides a first registration, and the registering of the 3D model and the endobronchially acquired image data provides a second registration. The first registration provides a coarse registration that is provided for finding a region of interest. The second registration provides a fine registration that is provided for finding the tip of the probe in the region of interest.

In an option, the result is fed back to fluoroscopy/CT registration to finetune the coarse step.

In an option, this is provided as a loop-like tuning process.

In an example, a proposition of registration methods for ultrasound image and the 3D model is provided. As an option, bronchoscopic imaging is provided and registered with the 3D model. The 3D model may be a CT-model, i.e. a model generated from CT image data.

In an example, it is also provided the step of:
- presenting the generated tracking information to a user.

In an example, the generated tracking information is presented on a display.

In an example, the tracking information comprises an overlay of an estimated position of the probe onto the 3D model.

In an example, the registering of the 3D model and the 2D X-ray projection data comprises transferring a position of the detected probe from a reference frame of the 2D X-ray projection data into a reference frame of the 3D model. The transferring of the position is based on the registration of the 3D model and the 2D X-ray projection data.

In an example, the tracking information comprises navigation information for the user for directing the probe towards the target location.

In an example, the 3D model is a subject-specific 3D model.

In an example, the tracking/localizing of the probe of the interventional device comprises determining a projection angle of the 2D X-ray projection in relation to the 3D model.

In an example, the registering of the 3D model and the 2D X-ray projection data comprises providing the 3D model with a first notation of a landmark and providing the 2D X-ray projection data with a second notation of the landmark, which first and second notation of the landmark are subject to an alignment.

In an example, "soft" alignment is provided.

In an example, the registering of the 3D model and the ultrasound image data (or the endobronchially acquired bronchoscopic image data) comprises generating a simulated ultrasound image (or simulated bronchoscopic image data) for a given position of the probe and comparing the simulated ultrasound image (or simulated bronchoscopic image) for similarity with the intraoperative ultrasound image data (endobronchially acquired intraoperative bronchoscopic image data).

In an example, the registering of the 3D model and the ultrasound image data (or the endobronchially acquired bronchoscopic image data) comprises extracting salient features from the ultrasound image data (or the endobronchially acquired bronchoscopic image data) and identifying these features in the 3D model.

In an example, both ultrasound image data and bronchoscopic image data are provided; and
wherein the bronchoscopic image data is registered with at least one of the group of the 3D model and the 2D X-ray projection data.

In an example, formulated mathematically, the workflow can be described as follows, where the inputs are:
I₀ = 3D model or CT image
Ix = projection X-ray (intraoperative)
I_{U} = ultrasound image (intraoperative)
The parameters to be optimized are:
*ρ*^{∗} = estimated probe position / pose
*Φ*₀ = 3D deformable transformation applied to I₀
The optimization can be performed using the method of coordinate ascent as follows:
1. ρ = *ƒ*ₜᵢₚ(I_{X})
2. φ₀ = argmin_{φ0} *ƒ*_{sim-xray}(Iₓ, I₀ ° φ₀)
3. ρ^{∗} = argmin_{ρ}∗ *ƒ*ₛᵢₘ₋ᵤₛ(Iᵤ, I₀ ° φ₀, p ° φ₀)
4. φ₀ = argmin_{φ0}*ƒ*_{sim-x-ray}(Iₓ, I₀ ° φ₀) + α|ρ^{∗} - ρ|²
5. Repeat steps 3 and 4 until convergence.

In step 1, *ƒ*ₜᵢₚ is a function that detects the ultrasound probe tip position from the x-ray image and p is the probe position estimated from projected X-ray.

In step 2, *ƒ*_{sim-xray} is a function that computes similarity between a projection X-ray and a 3D model transformed by φ₀.

In step 3, *ƒ*ₛᵢₘ₋ᵤₛ is a function that computes similarity between an ultrasound image and a 3D model given a particular probe position and pose.

In step 4, α is a weighting factor between the image similarity and the distance between landmarks ρ and ρ^{∗}. The solution to this problem is an optimization of φ₀ and ρ^{∗} given the above system of equations.

Workflow step 1 thus provides surgical tool tip detection. Workflow step 2 thus provides fluoroscopy-to-model registration, or projection to volume registration. In an example, steps 1 and 2 may comprise some inherent error in estimating the probe position, particularly step 2 which is a challenging problem (in which even a perfect solution does not solve the lack of depth information in projection X-ray). In order to rectify this expected error, the present solution provides for workflow steps 3 and 4. This allows an extraction of valuable information to improve probe location, as well as the integration of these systems into a single framework.

In an example of the method, as an update loop, when only one of the group of the X-ray projection data and the endobronchially acquired intraoperative image data is available, the current estimated probe position is updated only, using, in addition to the available image data, one of the group of the last known approximate region from the latest X-ray projection data and the latest endobronchially acquired intraoperative image data.

In an example of the method, before registering, it is provided the step of analyzing if both of the intraoperatively acquired 2D X-ray projection data of the area of interest of the subject and the endobronchially acquired intraoperative image data of at least a part of the area of interest of the subject are available; and both registration steps are provide if image data is available from sources.

In an example, a computer program or program element for controlling an apparatus according to one of the examples above is provided, which program or program element, when being executed by a processing unit, is adapted to perform the method steps of one of the method examples above.

As an example, the present solution can be used in interventional imaging systems that incorporate both intra-operative ultrasound with pre-operative 3D imaging and optionally, intra-operative 2D X-ray. Several clinical applications could benefit from the potential navigational assistance provided by this solution, including pulmonary lesion biopsy guided by EBUS/REBUS, intravascular diagnostic imaging guided by IVUS such as coronary artery stenting, trans-esophageal ultrasound guided procedures such as mitral valve replacement, etc.

It is provided an extraction of information from intraoperative endoscopic ultrasound to inform device position.

The co-registration of a pre-operative CT scan with real-time intraoperative imagery to detect the position or motion of a surgical device compensates the lack of information provided by intraoperative sources that for itself may be insufficient to completely determine the 3D device or tool position. As an example, intraoperative fluoroscopy provides a 2D projection image which leaves the depth dimension unresolved, leading to poor identifiability.

This is particularly the case for ultrasound - due to the intrinsic noise properties of ultrasound. The present solution describes an algorithmic approach for simultaneously incorporating information extracted from image processing performed on intraoperative EBUS/REBUS into a traditional lung nodule biopsy workflow.

In an example, a system for the use case involving ultrasound, fluoroscopy, and a pre-operative model is provided. The system comprises the following components and processing of the respective data:
A pre-operative 3D model of the individual patient's lungs or anatomy to be operated on is provided. This model could take the form of a point cloud, mesh, volumetric image (for instance, computed tomography), or otherwise.

Further, intermittent fluoroscopic or other projection x-ray imagery is provided acquired intraoperatively during device navigation.

Furthermore, intermittent EBUS/REBUS, or other endoscopic ultrasound imagery, is provided acquired intraoperatively during device navigation. Examples of such ultrasound probes include endobronchial ultrasound, radial endobronchial ultrasound, intravascular ultrasound and endoscopic/radial-endoscopic ultrasound for gastrointestinal procedures.

An image processing controller is provided that detects the real time probe tip position in intraoperative fluoroscopy or other projection X-ray imagery. Detection may be computed using any of the methods established in the art including but not limited to active-contour segmentation of the catheter and probe, threshold-based segmentation of the catheter and probe or neural network based object detection (e.g. YOLO, etc.).

Further, an image registration module is provided that receives as input a single pre-operative 3D model / image volume, e.g. CT or other volumetric imaging modality, and, when available, real time intraoperative X-ray images of the same anatomy with the ultrasound probe visible. As intermediate outputs, the module produces the projection angle at which the X-ray was acquired relative to the 3D model, as well as any deformation of the pre-operative 3D model that are visible in the intra-operative X-ray. As final output, the module produces the approximate location of the detected probe position relative to the 3D model (notably, with uncertainty in depth or even rather large error in depth). This module may incorporate any or more of the following components: A main registration submodule is provided that performs the intraoperative X-ray to preoperative model image registration. This may involve any or more of the following methods: gradient-based x-ray to CBCT projection similarity maximization; gradient-based x-ray to CT digitally reconstructed radiograph similarity maximization; and neural network based spatial transformer.

Further, a submodule is provided that receives as input two point landmarks, one in the 2D coordinate space of the projection X-ray and one in the 3D coordinate space of the pre-operative 3D model. The submodule considers the alignment of the two input landmarks as part of the objective function to be minimized in the optimization algorithm of the main registration submodule. When ultrasound imagery is available, this allows the feeding of information extracted by subsequent modules operating on ultrasound imagery to benefit intraoperative X-ray to preoperative model registration. Otherwise, the module operates without additional input from other image sources. This submodule may involve any or more of the following methods: gradient-based image and landmark registration by weighting image similarity and landmark distance; and neural network based spatial transformer taking landmarks and image volumes as input.

Further, an image registration module is provided that is configured to take as input a preoperative 3D model or image volume, real time 2D intra-operative ultrasound imagery, and optionally the approximate location of the ultrasound probe in the 3D space provided by the image processing controller above and the image registration module above. As output, this module produces the estimated location of the ultrasound probe in the coordinate space of the 3D model. The estimated location can be computed by one or more of the proposed methods.

In a first option, an image based comparison method is provided. Given the 3D structure of the anatomy in the approximate vicinity of the ultrasound probe tip, a simulated ultrasound image can be generated on demand for any given pose/position of the ultrasound probe tip. The simulated ultrasound image can then be compared for similarity to the true intra-operative image, where a highly similar image indicates an accurate position estimate. An optimization problem can be solved in the space of simulated ultrasound images where the objective to be minimized is a function of pose and position of the probe. This method would consist of the following submodules:
- In a first submodule, an ultrasound simulation submodule is provided. One or more existing methods can be used for the subtask of simulating ultrasound: physics based simulation; or neural network based generative models.
- In a second submodule, a simulated ultrasound similarity maximization submodule is provided, which changes the estimated probe position to maximize the similarity between the true image and the simulated image.

In a second option, a representation-based comparison method is provided. Given a real-time intraoperative ultrasound image, a compressed representation of the image is produced. Given a pre-operative 3D model or image volume and any estimated pose/position within the model, the same compressed representation of the image is produced in a supposed field of view of the ultrasound probe. Similar to the image registration module, the estimated pose/position of the probe is optimized based on maximizing the similarity between the compressed representations produced by intraoperative and preoperative imagery.

In a first example, the compressed representation may take the form of i) tissue based image segmentation in which each pixel is labelled by the estimated tissue type or properties. For instance, in the case of a CT image volume, the tissue type can be differentiated by the CT attenuation number. The compressed representation may also take the form of ii) an abstract representation such as a feature vector generated by a machine learning algorithm which may or may not be in the shape of an image.

In a second example, the method of computing the compressed representation may be based on i) an existing algorithm for image segmentation based on image contrast; or ii) a neural network based model that generates minimal equivalent representations from ultrasound and CT imagery.

In a third option, a feature based comparison method is provided. Given a real-time intraoperative ultrasound image, salient features are extracted from the image that provide more specific information about the probe position. These are generally features that can be easily identified in the pre-operative 3D model/image. Features to be extracted may take the form of airway/vessel bifurcations - a neural network based object detection algorithm has been designed to detect airway bifurcations. Given some estimate of probe motion from fluoroscopy or an image-based method operating on the real-time ultrasound and a history of detected bifurcations, comparison to the known bifurcations in the preoperative 3D model narrows down the possible locations of the probe and may eliminate uncertainty related to lack of depth information in fluoroscopy. Features to be extracted may also take the form of target lesion - an object detection algorithm can be personalized to a particular patient's lesion shape based on information from a pre-operative 3D model, such that this lesion is detectable in in intra-operative ultrasound.

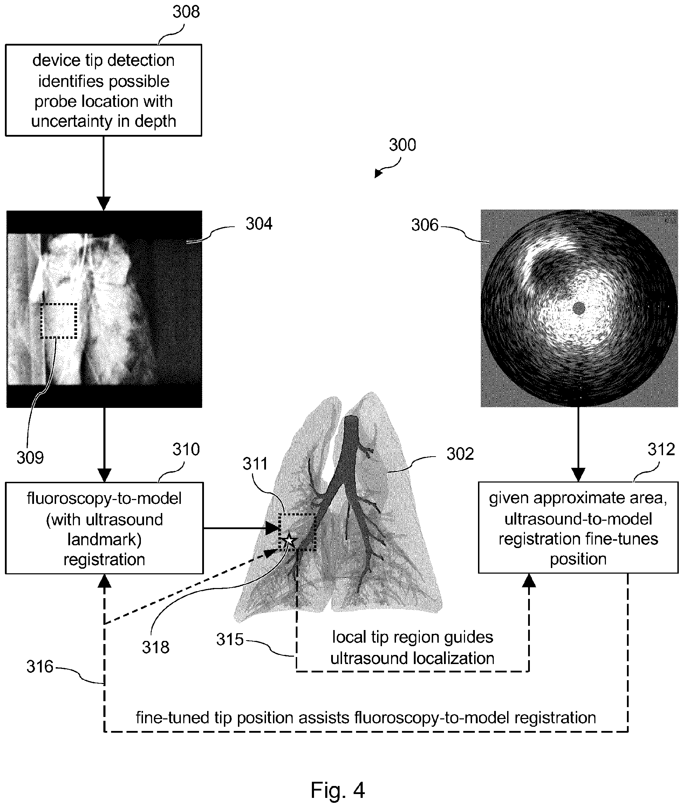

Further, an image processing controller is provided that coordinates the image registration modules above. When X-ray and ultrasound are both available, the controller executes the full algorithmic workflow of Fig. 4. When only X-ray is available, the controller updates the current estimated probe position by the module above only. When only ultrasound is available, the controller updates the current estimated probe position by the module described above only, using the last known approximate region from the latest X-ray image and the latest tracking information from the latest ultrasound image.

Further, a visualization controller is provided that receives as input the information extracted from the image registration module above and the image processing controller above and overlays the fine-tuned estimated position of the ultrasound probe onto the pre-operative 3D model and displays to the user where in the 3D model they are currently located, relative to the target location. Alternatively, the visualization controller can augment the fluoroscopy visualization by taking the estimated position of the ultrasound probe relative to the target location and displaying information to the user to direct them towards the optimal path/direction.

In another example, a system is provided for the use case involving bronchoscopy, ultrasound, fluoroscopy, and a pre-operative model. In an alternative embodiment, intraoperative ultrasound and bronchoscopy imaging are performed simultaneously. This covers the use case where the target lesion is at a location that can be reached by both bronchoscope and ultrasound probe and where bronchoscope-less navigation is not being considered. This example includes an additional image registration module that interacts with the modules from the embodiments above to further refine the ultrasound/bronchoscope tip location in reference to the 3D model. Information can be extracted from the bronchoscope in a number of existing and proposed methods.

Fig. 4 shows an example of an algorithmic workflow. As input data, the proposed framework takes a single pre-operative 3D model 302 or image generated from CT imaging and real-time intra-operative fluoroscopy 304 and ultrasound imagery 306. One option provides the following steps:
- Image processing 308 is performed to locate the ultrasound probe tip in fluoroscopy. For example, the device tip detection identifies possible probe location with uncertainty in depth. A frame 309 indicates the area of the probe tip.
- Fluoroscopy-to-model or fluoroscopy-to-CT image registration 310 is performed to place the approximate probe position in the coordinate space of the 3D model.
- Given the ultrasound image and the approximate probe position 311, an algorithm 312 uses the image information and compares to the 3D model to refine the probe position. A local tip region is used 315 to guide ultrasound localization.
- The refined probe position is combined 314 with the detected tip from step 308 and used as a landmark 316 to improve fluoro-to-model registration.
- As an option, steps 2 to 4 are repeated until convergence and as necessary as new intraoperative images come in.

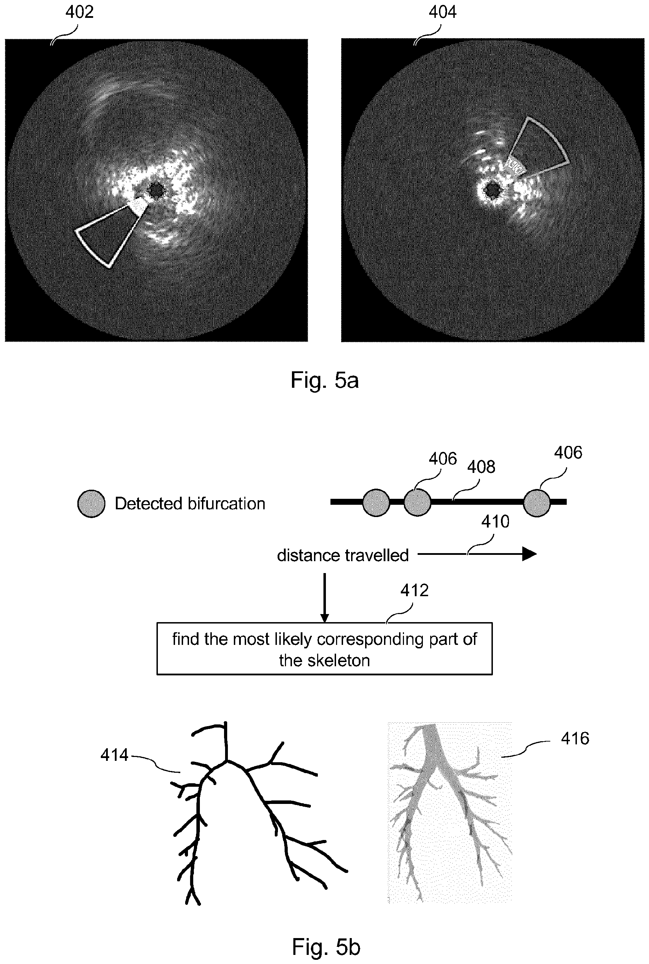

Fig. 5a and 5b show an example for bifurcation detection framework. Two examples are shown of a neural network object detection model trained on intravascular ultrasound imagery collected from a preserved swine lung phantom. The examples illustrate the ability of the method to differentiate bifurcations (a small-angle loss of acoustic contact with the airway wall) with and without the presence of abnormalities such as lesions.

Fig. 5a shows two examples of bifurcation detection using a neural network object detection model in the presence of a lesion 402 and without 404.

Fig. 5b shows results of steps of a workflow. Bifurcations 406 are detected as the probe travels on a path 408. A travel distance 410 is determined and provided to a step 412 of finding the most likely corresponding part of the airway tree structure 414, by a search of possible trajectories and comparison to the expected bifurcation pattern. The airway tree structure can be extracted by segmentation and centerline extraction of the airways 416 from the pre-operative 3D model. The travel distance 410 may be determined by a combination of the distance the probe has advanced and the probe tracking from intraoperative X-ray.

The term "subject" may also be referred to as individual. The "subject" may further also be referred to as patient, although it is noted that this term does not indicate whether any illness or disease is actually present with the subject.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit or be distributed over more than one computer units, which might also be part of an embodiment of the present invention. This computing unit may be adapted to perform or induce a performing of the steps of the method described above. Moreover, it may be adapted to operate the components of the above described apparatus. The computing unit can be adapted to operate automatically and/or to execute the orders of a user. A computer program may be loaded into a working memory of a data processor. The data processor may thus be equipped to carry out the method of the invention.

Aspects of the invention may be implemented in a computer program product, which may be a collection of computer program instructions stored on a computer readable storage device which may be executed by a computer. The instructions of the present invention may be in any interpretable or executable code mechanism, including but not limited to scripts, interpretable programs, dynamic link libraries (DLLs) or Java classes. The instructions can be provided as complete executable programs, partial executable programs, as modifications to existing programs (e.g. updates) or extensions for existing programs (e.g. plugins). Moreover, parts of the processing of the present invention may be distributed over multiple computers or processors.

As discussed above, the processing unit, for instance a controller implements the control method. The controller can be implemented in numerous ways, with software and/or hardware, to perform the various functions required. A processor is one example of a controller which employs one or more microprocessors that may be programmed using software (e.g., microcode) to perform the required functions. A controller may however be implemented with or without employing a processor, and also may be implemented as a combination of dedicated hardware to perform some functions and a processor (e.g., one or more programmed microprocessors and associated circuitry) to perform other functions.

Examples of controller components that may be employed in various embodiments of the present disclosure include, but are not limited to, conventional microprocessors, application specific integrated circuits (ASICs), and field-programmable gate arrays (FPGAs).

This exemplary embodiment of the invention covers both, a computer program that right from the beginning uses the invention and a computer program that by means of an up-date turns an existing program into a program that uses the invention.

Further on, the computer program element might be able to provide all necessary steps to fulfil the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section. A computer program may be stored and/or distributed on a suitable medium, such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated, and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfil the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. A device (10) for endobronchial probe tracking, the device comprising:
- a data input (12);
- a data processor (14); and
- an output interface (16);
wherein the data input is configured: to provide a 3D model of an area of interest of a subject; to provide intraoperatively acquired 2D X-ray projection data of the area of interest of the subject; and to provide endobronchially acquired intraoperative image data of at least a part of the area of interest of the subject;
wherein the data processor is configured: to track a probe of an interventional device, which probe is acquiring the image data, in the 2D X-ray projection data; to register the 3D model and the 2D X-ray projection data; to register the 3D model and the endobronchially acquired intraoperative image data; and to generate tracking information for the probe of the interventional device in relation to a target location within the area of interest; and
wherein the output interface is configured to provide the generated tracking information.

2. Device according to claim 1, wherein the provided endobronchially acquired intraoperative image data is provided as at least one of the group of
i) endobronchially acquired intraoperative ultrasound image data; and
ii) endobronchially acquired intraoperative bronchoscopic image data.

3. Device according to claim 1 or 2, wherein the data processor is configured to analyze, before registration, if both of the intraoperatively acquired 2D X-ray projection data of the area of interest of the subject and the endobronchially acquired intraoperative image data of at least a part of the area of interest of the subject are available; and the data processor is configured to provide both registration steps only if image data is available from sources.

4. Device according to claim 1, 2 or 3, wherein the tracking information comprises at least one of the group of:
- an overlay of an estimated position of the probe onto the 3D model; and
- navigation information for the user for directing the probe towards the target location.

5. Device according to one of the preceding claims, wherein the 3D model is a subject-specific 3D model.

6. Device according to one of the preceding claims, wherein, to track the probe of the interventional device, the data processor is configured to determine a projection angle of the 2D X-ray projection in relation to the 3D model.

7. Device according to one of the preceding claims, wherein, to register the 3D model and the 2D X-ray projection data, the data processor is configured: to provide the 3D model with a first notation of a landmark; and to provide the 2D X-ray projection data with a second notation of the landmark, which first and second notation of the landmark are subject to an alignment.

8. Device according to one of the preceding claims, wherein, to register the 3D model and the endobronchially acquired image data, the data processor is configured: to generate, based on the 3D model, a simulated image for a given position of the probe; and to compare the simulated image for similarity with the intraoperative image data.

9. Device according to one of the preceding claims, wherein, to register the 3D model and the image data, the data processor is configured: to extract salient features from the image data; and to identify these features in the 3D model.

10. Device according to one of the preceding claims, wherein the data input is configured to provide both ultrasound image data and bronchoscopic image data; and
wherein the data processor is configured to register the bronchoscopic image data with at least one of the group of the 3D model and the 2D X-ray projection data.

11. Device according to one of the preceding claims, wherein, as an update loop, when only one of the group of the X-ray projection data and the endobronchially acquired intraoperative image data is available, the data processor is configured to update the current estimated probe position only, by use of, in addition to the available image data, one of the group of the last known approximate region from the latest X-ray projection data and the latest endobronchially acquired intraoperative image data.

12. A system (50) for bronchial intervention procedures, the system comprising:
- a 2D X-ray imaging arrangement (52);
- an imaging arrangement (60) for endobronchial imaging; and
- a device for endobronchial probe tracking (10) according to one of the preceding claims;
wherein the 2D X-ray imaging arrangement comprises an X-ray source and an X-ray detector configured to generate the 2D X-ray projection data; and
wherein the imaging arrangement comprises an interventional device with an imaging probe for insertion into the bronchial pathway configured to generate the intraoperative image data.

13. A method (200) for tracking of an endobronchial probe, the method comprising the following steps:
- providing (202) a 3D model of an area of interest of a subject;
- providing (204) intraoperatively acquired 2D X-ray projection data of the area of interest of the subject;
- providing (206) endobronchially acquired intraoperative image data of at least a part of the area of interest of the subject;
- tracking (208) a probe of an interventional device, which probe is acquiring the image data, in the 2D X-ray projection data;
- registering (210) the 3D model and the 2D X-ray projection data;
- registering (212) the 3D model and the endobronchially acquired intraoperative image data;
- generating (214) tracking information for the probe of the interventional device in relation to a target location within the area of interest; and
- providing (216) the generated tracking information.

14. A computer program enabling a processor to carry out the method of claim 13.

15. A computer readable medium having stored the program element of claim 14.
